# EUROPEAN PATENT SPECIFICATION

(11) **EP 3 275 434 B1**
(45) Date of publication and mention of the grant of the patent: **26.08.2020**
(21) Application number: 16181467.8
(22) Date of filing: 27.07.2016
(51) Int. Cl.: A61K 9/22, A61K 31/4184

(54) **AN EXTENDED RELEASE ORAL DOSAGE FORM**
ORALE DOSIERFORM MIT VERLÄNGERTER FREISETZUNG
FORME POSOLOGIQUE ORALE À LIBÉRATION PROLONGÉE

(43) Date of publication of application: 31.01.2018
(73) Proprietor: SANDOZ AG, 4056 Basel (CH)
(72) Inventor: RANEBURGER, Johannes, 6250 Kundl (AT); WIEDMANN, Markus, 83607 Holzkirchen (DE); SCHWARZ, Franz Xaver, 6250 Kundl (AT)
(74) Representative: Prüfer & Partner mbB Patentanwälte · Rechtsanwälte

(56) References cited:
- WO-A1-2017/136330
- CN-A- 1 733 305
- MOSTAFA NAEL M ET AL: "A phase 1 study to evaluate effect of food on veliparib pharmacokinetics and relative bioavailability in subjects with solid tumors", CANCER CHEMOTHERAPY AND PHARMACOLOGY, SPRINGER VERLAG, BERLIN, vol. 74, no. 3, 23 July 2014 (2014-07-23), pages 583-591, XP035379519, ISSN: 0344-5704, DOI: 10.1007/S00280-014-2529-2 [retrieved on 2014-07-23]
- LANDRUM LISA M ET AL: "A phase I trial of pegylated liposomal doxorubicin (PLD), carboplatin, bevacizumab and veliparib in recurrent, platinum-sensitive ovarian, primary peritoneal, and fallopian tube cancer: An NRG Oncology/Gynecologic Oncology Group study", GYNECOLOGIC ONCOLOGY, ACADEMIC PRESS, LONDON, GB, vol. 140, no. 2, 23 November 2015 (2015-11-23), pages 204-209, XP029388377, ISSN: 0090-8258, DOI: 10.1016/J.YGYNO.2015.11.024

## Description

### FIELD OF THE INVENTION

The present invention relates to an extended release oral dosage form comprising 2-((R)-2-Methylpyrrolidin-2-yl)-1H-benzimidazole-4-carboxamide (veliparib) and a gel-forming polymer. The invention further relates to processes for preparing the extended release formulation, to the extended release formulation as a medicament for the prevention and/or treatment of cancers and to a dosage regimen for the extended release formulation for the treatment or prevention of cancers.

### BACKGROUND OF THE INVENTION

PARP is an important protein in DNA repair pathways, especially in the base excision repair, which is involved in repairing DNA with single strand breaks. By inhibiting poly(ADP-ribose) polymerase (PARP), single strand breaks can accumulate, eventually turning into double stand breaks. Some cancer cells with a defective homologous recombination pathway, like BRCA-deficient cell lines, are susceptible to the impairment of PARP, and inhibitors of PARP can preferentially kill such cancer cells over normal cells. Also, PARP inhibitors increase the cytotoxicity of chemotherapies that induce single strand breaks.

Veliparib is the INN for 2-((R)-2-Methylpyrrolidin-2-yl)-1H-benzimidazole-4-carboxamide. It inhibits PARP-1 and PARP-2 at nanomolar concentrations. Veliparib is administered orally. In vitro, veliparib decreases PAR levels in irradiated cells, reduces survival of cell clones, inhibits DNA repair, and increases apoptosis.

Pharmaceutical compositions of veliparib are broadly disclosed in WO 2006/110816 on page 30 and in WO 2009/049111 on page 10. Though there are no concrete examples, in clinical studies veliparib is dosed twice daily at 400 mg. As a consequence of the 40mg dose of veliparib per capsule, a patient has to swallow ten capsules twice a day. This dosage regimen has been recommended as being safe and efficacious for BRCA-1 and BRCA-2 mutated platinum-refractory ovarian tumors. The twice daily dosage regimen is consistent with an immediate release formulation of a BCS-class 1 drug which is resorbed quickly and which has a short elimination half-life of about 6 hours. When veliparib was administered as an immediate release tablet at 2 x 400 mg per day, side effects like anemia, fatigue, nausea, pain and cough were reported, leaving room for the improvement of how veliparib is to be administered.

Landrum et al., Gynecologic Oncology 140 (2016): 204-209, discloses a phase I trial in which veliparib capsules are administered twice daily.

### BRIEF DESCRIPTION OF THE INVENTION

The present invention provides an extended release oral dosage form comprising veliparib and a gel-forming polymer for the treatment of cancer, wherein the extended release dosage form is to be administered twice daily. The dosage form enables effective PARP-inhibition at a low daily dose and/or with few individual dosage forms to be administered per day. The extended release formulations of the present invention also enable effective treatment of a cancer that is sensitive to PARP-1/2-inhibition with a lower incidence of side effects, for example when compared to veliparib immediate release capsules 400mg twice daily (800mg per day).

The present invention also relates to processes for the manufacture of the extended release oral dosage form. The present invention thus reduces the pill burden for patients and/or the drug burden and/or side effects, while at the same time providing an effective treatment of veliparib-sensitive cancer.

### DESCRIPTION OF THE FIGURES

Figure 1 - Time-Concentration curve showing the blood plasma concentration of veliparib against time. Veliparib is administered as ten individual 40 mg IR capsules (400 mg total / 800mg total per day) twice a day. The Cₘₐₓ/Cₘᵢₙ ratio is about 7.
Figure 2 *-In -vitro* dissolution profile of a Veliparib 80 mg ER tablet prepared in accordance with example 1. The ER tablet is tested in 3 different dissolution media *viz.* water (Figure 2.1), 0.1N HCl (Figure 2.2) and acetate buffer of pH 4.5 (Figure 2.3) using dissolution type II (paddle) apparatus at 50 RPM.

### DEFINITIONS:

Veliparib (2-((R)-2-Methylpyrrolidin-2-yl)-1H-benzimidazole-4-carboxamide) as used herein means veliparib as a free base, a pharmaceutically acceptable salt and/or a hydrate or a solvate or veliparib dihydrochloride salt. If quantities are indicated, these quatities relate to the amount of veliparib free base, regardless of whether veliparib is used as its free base form or a salt, solvate or hydrate form.

The term 'extended release oral dosage form' shall mean any oral dosage form, which releases the active ingredient at rates, which are sufficient to provide periods of prolonged therapeutic action following each administration of a single dose of such a dosage form. An extended release oral dosage form typically comprises one or more release-modifying agents, which change a medicinal product's drug-release pattern to achieve the desired drug plasma profile for a given time. The majority of release-modifying agents are polymers that differ in solubility, ease of erosion, rate of swelling, or sensitivity to the biological environment in which they are placed. These polymers have been used to fabricate matrix- or membrane-based drug delivery systems for oral, parenteral, transdermal, and other routes of administration. Matrix controlled-release drug delivery systems can be classified as hydrophilic eroding matrices, hydrophilic noneroding matrices, or hydrophobic matrices. In membrane controlled-release drug delivery systems the drug reservoir is coated by a rate-controlling polymeric membrane that may consist of a blend of polymers to control release.

The term peak plasma concentration is Cₘₐₓ in blood plasma observed after administration. The term trough plasma concentration is Cₘᵢₙ in blood plasma observed after administration and just prior to the administration of a subsequent dose in a given dosage regimen. The veliparib plasma concentration can be determined according to the approach described in Mostafa et al., Cancer Chemother Pharmacol (2014) 74: 583-591. Pharmacokinetic terms have the typical meaning as recognized in the art and in case of doubt are to be interpreted according to the European Pharmacopoeia Version 8.8.

The Biopharmaceutical Classification System as used by the FDA on July 1 2016 is relevant for the terms 'good soluble' or 'highly soluble' or "BCS class1".

The term peak-to-trough variations is the ratio of the maximum or "peak" plasma concentration (Cₘₐₓ) of a drug observed after its administration to the minimum or "trough" plasma concentration (Cₘᵢₙ) of a drug observed after its administration and just prior to the administration of a subsequent dose in a given dosage regimen.

The term steady state concentration Cₛₛ is the concentration of a drug or chemical in a body fluid - usually plasma - at the time a "steady state" has been achieved, i.e. rates of drug administration and drug elimination are about equal.

The term "Crystalline" as used herein defines a solid material whose constituent atoms are distributed in an ordered and repetitive pattern in a long-range positional order. Long-range order e.g. extends over approximately at least 1000 atoms. In powder X-ray diffraction a crystalline material will typically show characteristic reflection patterns.

The USP paddle method and the paddle apparatus are as defined in http://www.usp.org/sites/default/files/usp_pdf/EN/USPNF/2011-02-25711DISSOLUTION.pdf

The term "dissolution time" is the time required for a given amount (or fraction) of veliparib to be released into solution from a solid dosage form in a dissolution experiment using acetate buffer pH 4.5 and the USP paddle method and paddle apparatus. Dissolution time is measured in vitroin experiments in which the amount of veliparib in solution is determined as a function of time.

The term AUC is defined as area under the curve, *i*.*e*.the area under the plot of the plasma concentration of the drug (linear presentation of the concentration) against time after drug administration. The area is conveniently determined by the "trapezoidal rule".

Dose as used herein is the quantity of drug, or dosage form, administered to a subject at a given time.

The term "viscosity" is defined as per PhEur monograph: 2.2.8. and viscosity is determined by Capillary viscometer method as per PhEur monograph: 2.2.9.

### DETAILED DESCRIPTION OF THE INVENTION

The present invention provides an extended release dosage form of veliparib. The extended release dosage form of present invention is to be administered for at least seven days and the peak plasma concentration at day 7 is at most five times as high as the trough plasma concentration at day 7. It is recommend that the extended release dosage form be administered at least every 12 hours.

Veliparib has a short elimination half-life of about 6 hours. Given this short half-life immediate release tablets of velaparib have been administered twice daily at 400 mg per administration in clinical studies. This dose of 400 mg is provided in the form of ten individual 40 mg capsules. Consequently a patient has to swallow ten capsules twice a day.

The extended release dosage form of the present invention for veliparib achieves to lower the overall daily dose of Veliparib. Moreover patients no longer have to swallow 20 capsules over a period of 24 hours.

Besides, the extended release dosage form of the present invention, in particular in combination with an at least twice daily administration, achieves lower fluctuations in Veliparib blood plasma levels upon administration when compared to the above described dosage regimen for veliparib immediate release, for example when the extended release dosage form of the present invention is administered in multiples of 2-5 tablets twice a day.

BCS class I drugs, such as veliparib, show high solubility and high permeability. Such drugs, owing to their solubility and/or permeability, are quickly absorbed in blood plasma and are quickly eliminated. Such drugs can show a sharp rise and quick fall in blood plasma concentrations, leading to side effects resulting from such fluctuations. This can become problematic if, during a long-term treatment, the trough concentration of the drug needs to be kept above a certain minimal value so as to assure sustained effectiveness of the treatment, while a high peak concentration might cause side effects.

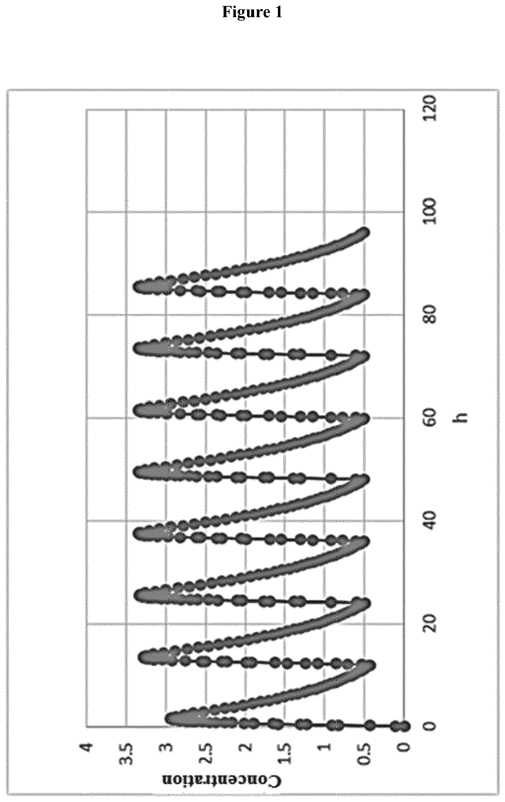

The present inventors have realized that the *In-vivo* plasma concentration profile of veliparib when administered as immediate release dosage forms at 400 mg BID, can be such that the blood plasma fluctuations are quite high. The Cₘₐₓ almost reaches 3.5 µg/ml whereas Cₘᵢₙ is just around 0.5 µg/ml. This is shown in Figure 1. Thus, undesireably high peak concentrations are obtained in order to keep the trough concentration above a therapeutic threshold. In contrast, the extended release dosage form of the present invention enables the provision of therapeutically effective trough concentrations at all times without attaining such a high peak concentration. Moreover, when the extended release dosage form of the present invention is administered twice daily, even an overall dose reduction can be obtained while at the same time keeping the through concentration above the therapeutic threshold.

The present invention therefore relates to an extended release dosage form comprising an effective amount of veliparib, as defined in the claims, for the treatment of a cancer which is sensitive to PARP-inhibition, wherein the extended release dosage form is to be administered for at least seven days and wherein the peak plasma concentration at day 7 is at most five times as high as the trough plasma concentration at day 7, when said extended release dosage form is administered every 12 hours. Preferably the extended release dosage form of the present invention is to be administered for at least seven days, and at most twice daily, such as once daily or twice daily.

The amount of veliparib to be administered per day is preferably at most 750mg, such as from 300mg to 750mg or from 500mg to 700mg. The trough plasma concentration at day 7 is preferably at least 400ng veliparib / ml plasma. The peak plasma concentration at day 7 is preferably at most 3.5 times as high as the trough plasma concentration at day 7, when said extended release dosage form is administered every 12 hours, more preferably the peak plasma concentration at day 7 is 2.0 times to 3.3 times as high as the trough plasma concentration at day 7.

The skilled person is aware that the amount of gel forming polymer required for achieving an extended release effect is dependent on the nature of the gel forming agent. For example, low solubility ethyl cellulose produces effective extended release tablets of Veliparib in which the Veliparib : polymer ratio is from 1:0.05 to 1:0.5, whereas soluble gel formers such as HPMC or polyethylene oxides are effectively used in a drug: polymer ratio of 1:0.5 to 1:10, preferably in a drug: polymer ratio of 1:0.5 to 1:5.

The extended releases solid oral dosage form of the present invention optionally comprises further excipients. The optionally further pharmaceutically acceptable excipients can be selected from binders, lubricants, release modifying agents and flow condition agents.

Veliparib can be employed as the free base, a pharmaceutically acceptable salt, a hydrate or a solvate. Veliparib free base is the preferred form of veliparib to be used, in particular crystalline veliparib free base. Another preferred form of veliparib is a veliparib hydrochloric acid salt, such as a veliparib dihydrochloride and in particular crystalline veliparib dihydrochloride. The amount of Veliparib in the extended release dosage form of the present invention is preferably from 50 mg to 250 mg, preferably from 80 mg to 240 mg, such as from 80 mg to 160 mg. Preparation of a 80 mg tablet is described in the exemplifying section. Other dosage forms containing 120 mg, 160 mg, 200 mg and 240 mg of Veliparib can be prepared by proportionately adjusting the inactive ingredients as understood by a person skilled in the art. As veliparib amounts are given for veliparib free base, the skilled person will appreciate that quantities of diluents are to be adjusted whenever a form of veliparib is used having a higher molecular weight than veliparib free base.

A preferred gel-forming polymer is selected from the group of hydroxymethyl cellulose, hydroxypropyl cellulose, hydroxyethyl cellulose, hydroxypropyl methylcellulose, methyl cellulose, ethyl cellulose, polyvinylpyrrolidone, polyethylene glycols, polyethylene oxide and poloxamers, and as the gel-forming polymer hydroxypropyl methylcellulose (HPMC) is particularly preferred. The viscosity of the HPMC should be between 3000 and 100000 mPa.s (cP), preferably 7500 and 100000 mPa.s (cP), and most preferably 11250 and 100000 mPa.s (cP) (viscosities are provided for a 2% (w/w) aqueous solution at 20°C).

The viscosity of 2% aqueous solution at 20°C is determined according to:
a) PhEur (PhEur monograph: Hypromellose, 2.2.8. Viscosity, and 2.2.9. Capillary viscometer method).

The substitution degree for methoxy groups of this cellulose is preferably from 19 to 30 % by weight, such as from 19 to 28%, for example from 19 to 24 % by weight, and the substitution degree of hydro xypropoxy groups is preferably from 4 to 12% by weight, such as from 7 to 12% by weight. It is preferred that HPMC is a mixture of HPMC of various grades comprising viscosities of 15 mPa.s (cps), 15000 mPa.s (cps), 100000 mPa.s (cps).

The further inactive ingredients that can be used in the extended release dosage forms apart from the gel forming polymers include release modifying agents, binders, lubricants, and/or flow condition agents.

The release modifying agent is preferably selected from the group of lactose, mannitol, sorbitol, calcium phosphate, aluminum silicate, paraffin, carboxypolymethylene, carboxyvinyl polymer, acrylic acid polymer, ethyl cellulose and polyethylene glycol and any combination thereof.

The binder is preferably selected from the group of hydroxypropyl cellulose, microcrystalline cellulose, polyvinylpyrrolidone, gelatine, polyethylene glycol, glycerylbehenate, glycerylmonostearate, carnauba wax and any combination thereof. Preferably the amount of binder in the composition is from 0 to 45 % w/w. The ratio of binder to active substance may be from 1:1 to 1:10, preferably from 1:2 to 1:7.

The lubricant is preferably selected from the group of magnesium stearate, calcium stearate, sodium stearyl fumarate, stearic acid, polyethylene glycol, talc and any combination thereof.

The flow condition agent is preferably selected from the group of colloidal silicon dioxide, talc and combination thereof.

The extended release dosage form may be coated or uncoated. In one preferred embodiment to extended release dosage form is coated. The coating layer preferably comprises a polymer such as ethyl cellulose, hydroxypropyl cellulose, polyethylene glycol, low viscosity HPMC, acrylic and methacrylic esters, ethyl acrylate and methyl methacrylate or mixtures thereof. The coating layer may further comprise plasticizers, colouring agents, taste-masking agents and/or anti-adhesion agents.

The extended release dosage forms of the present invention can exhibit a specific *In-vitro* dissolution pattern when measured according to the USP paddle method as described above.

| Time points | Time in hrs | % Cumulative Release |
|---|---|---|
| 1 | 1 hr | Less than 50 %, preferably less than 40 % |
| 2 | 3 hrs | Less than 75 %, preferably less than 70 % |
| 3 | 6 hrs | Less than 95 %, preferably less than 90 % |

The above release profile indicate *In-vitro* dissolution profiles of preferred extended release dosage forms of present invention. It is desired that also a minimum amount of Veliparib be released at each stated point in time. The extended release Veliparib dosage form of the present invention thus preferably exhibits the following minimal values in dissolution profiles when measured according to the USP paddle method as described above, at the stated time points as follows:

| Time points | Time in hrs | % Cumulative Release |
|---|---|---|
| 1 | 1 hr (60 min) | More than 5%, preferably more than 10 % |
| 2 | 3 hrs (180 min) | More than 25 %, preferably more than 40 % |
| 3 | 6 hrs | More than 50% preferably more than 60 % |

In a preferred embodiment, the extended release dosage form of the present invention exhibits the following In-vitro release profile

| Time points | Time in hrs | % Cumulative Release |
|---|---|---|
| 1 | 1 hr | Around 25 - 30 % |
| 2 | 3 hrs | Around 55 - 60 % |
| 3 | 6 hrs | Around 75 - 80 % |

In yet another preferred embodiment, the extended release dosage form according to the present invention exhibits an in-vitro release in the USP paddle method of about 53.5% Veliparib after 180 min and about 76.7 % after 360 min. This is shown in Figure 4.

The dosage form of the invention may comprise a coating layer. This coating layer may optionally have an extended release function and can contain additives for the modification of the release of the pharmaceutically active substance.

Suitable polymers that can be used in the coating layer are ethyl cellulose, hydroxypropyl cellulose, hydroxypropyl methylcellulose, polyethylene glycol, acrylic and methacrylic esters such as Eudragit RL and RS (Rohm Pharma). Ethyl acrylate and methyl methacrylate such as Eudragit NE (Rohm Pharma) may also be used in the coating layer. The coating layer may further comprise binders such as microcrystalline cellulose, hydroxypropyl cellulose and the like, plasticizers such as polyethylene glycol, acetyl tributyl citrate and the like and colour agents such as titanium dioxide, iron oxide and the like. Also, antiadhesion agents such as colloid silicon dioxide, talc and the like may be used in the coating layer. The coating layer may additionally comprise taste-masking agents. As coating fluid may be used water or alcohols such as ethanol, optionally containing antibacterial agents such as hydrogen peroxide. The coating layer may be applied by way of spray coating in a fluidised bed, pan-coating or another coating technique known to a person skilled in the art. The extended release dosage form of the present invention is preferably coated.

The following processes may be used to prepare an extended release dosage form in accordance with the present invention:
1) Dry mixing with gel forming polymer - preferred gel former Polyethylene oxide
2) Granulation or spray granulation with gel forming polymer - preferred gel former HPMC or ethyl cellulose
3) Compaction with gel forming polymer - preferred gel former Polyethylene oxide
4) Melt extrusion with gel forming polymer - preferred gel former poloxamer
5) Coating of tablet with gel forming polymer - preferred gel former ethyl cellulose or ethyl acrylate methyl methacrylate in combination with HPMC, PVP or other water soluble pore formers.

### Dry Mixing

Processes for the manufacture of an extended release dosage form according to present invention may comprise the following steps:
i) mixing the veliparib with the gel-forming polymers, release modifying agent and optionally binders, lubricants, modifying agents and other excipients to obtain a powder mixture,
ii) forming the obtained powder mixture into a suitable solid dosage form and
iii) optionally coating the obtained dosage form.

### Spray Granulation

Processes for the manufacture of an extended release dosage form according to present invention may comprise the following steps:
i)
   a. mixing the veliparib with the gel-forming polymers, release modifying agent and optionally binders and other excipients,
   b. sieving and blending the mixture,
   c. spray granulating said mixture,
   d. optionally drying the obtained granulate,
   e. adding lubricant to the granulate of step d) and sieving,
   f. compressing the granulate powder mass of step e),
ii) forming the obtained dry powder mixture into a suitable solid dosage form and
iii) coating the obtained dosage form.

### Granulation

Processes for the manufacture of an extended release dosage form according to present invention following steps:
i) mixing the veliparib with the gel-forming polymers, release modifying agent and optionally binders and other excipients,
ii) granulating said mixture,
iii) optionally drying the obtained granulate,
iv) mixing the granulate with other excipients to obtain a powder mixture,
v) forming the obtained dry powder mixture into a suitable solid dosage form and
vi) optionally coating the obtained dosage form.

### Granulation followed by Compaction

Processes for the manufacture of an extended release dosage form according to present invention may comprise the following steps:
i) mixing the veliparib with the gel-forming polymers, release modifying agent and optionally binders and other excipients,
ii) granulating said mixture,
iii) optionally drying the obtained granulate,
iv) compressing the granulate powder mass into loose compacts,
v) milling the compacts and mixing them with other excipients to obtain a powder mixture,
vi) forming the obtained dry powder mixture into a suitable solid dosage form and
vii) optionally coating the obtained dosage form.

### Compaction

Processes for the manufacture of an extended release dosage form according to present invention may comprise the following steps:
i) mixing the veliparib with the gel-forming polymers, release modifying agent and optionally binders and other excipients,
ii) compressing the dry mixed mass into loose compacts,
v) milling the compacts and mixing them with other excipients to obtain a powder mixture,
vi) forming the obtained dry powder mixture into a suitable solid dosage form and
vii) optionally coating the obtained dosage form.

### Melt extrusion

Processes for the manufacture of an extended release dosage form according to present invention may comprise the following steps:
i) mixing the veliparib with the gel-forming polymers, and optionally release modifying agent and binders and other excipients,
ii) subjecting the mixture to melt extrusion
iii) drying the extrudates
iv) sieving / milling the extrudates
v) mixing the milled granules with extragranular component to obtain blend
vi) forming the obtained blend into a suitable solid dosage form and
vii) optionally coating the obtained dosage form.

### Tablet coating

Processes for the manufacture of an extended release dosage form according to present invention may comprise the following steps:
i) mixing the veliparib with release modifying agent and optionally binders and other excipients,
ii) subjecting the mixture to granulation / spray granulation / compaction
iii) drying the granulate
iv) sieving / milling the extrudates
v) mixing the milled granules with extragranular component to obtain blend
vi) forming the obtained blend into a suitable solid dosage form and
vii) coating the obtained dosage form with gel forming agent selected from the group consisting of ethyl cellulose and ethyl acrylate methyl methacrylate to obtain extended release dosage form.

The dosage form may be prepared by embedding the active substance in a mixture of gel forming agent and release modifying agent.

Alternatively, the dosage form may be prepared from a granulated powder. The homogeneous powder may be obtained by mixing the active substance, the gel-forming polymers and optionally excipients such as binders in a suitable mixer. Then the mixture may be granulated in a mixer. The granulation can be performed in water or another granulation liquid such as an alcohol, e.g. ethanol, methanol, isopropanol, a ketone, e.g. acetone or aqueous mixtures thereof. From an environmental point of view water is preferred. The combination of PVP and HPMC in the ratio of 1 to 3, as used in one of the embodiments of the present invention, makes it feasible to use water as the granulation liquid instead of an organic liquid. The resultant wet granulation may thereafter be dried in a drying cabinet or in a fluid bed dryer and milled through a screen. The granulation may also be performed at elevated temperatures by using meltable binders. The cooled granulation may be milled through a screen. The dry granulate powder mass is then mixed with the remaining excipients and compressed into a suitable dosage form.

Moreover, the dosage form may also be prepared by first compressing the dry granulate powder mass into loose compacts. These loose compacts may be milled through a screen and finally mixed with other excipients such as binders, lubricants and flow condition agents. The dry, homogeneous powder mass may then be manufactured into a suitable dosage form, e.g. compressed into tablets in a tablet machine. Other suitable oral dosage forms are capsules, mini-tablets and the like.

The extended release oral dosage form composition according to the invention may be used in the prevention and/or treatment of cancer, for example for the prevention and/or treatment of cancers related to but not limiting to bladder, breast, colorectal, liver, lung, Non-Small Cell, Lymphoma, Non-Hodgkin's, ovarian, prostate and unspecified solid tumor, in particular breast neoplasms, carcinoma, hepatocellular carcinoma, Non-Small-Cell Lung cancer, colorectal Neoplasms, Liver Cancer; Liver Neoplasms; Lymphoma; Lymphoma, Non-Hodgkin; Neoplasms, Unknown Primary; Ovarian Cancer; Ovarian Neoplasms; Prostatic Neoplasms; Urinary Bladder Neoplasms.

The extended release oral dosage form of the invention may be used in the prevention and/or treatment of diseases and disorders and disturbances which are susceptible of PARP 1 and PARP 2 inhibitors. The extended release oral dosage form composition according to the invention may thus be used in the prevention and/or treatment of Adamantinoma; Astrocytoma; Brain Neoplasms; Choroid Plexus Neoplasms; Craniopharyngioma; Ependymoma; Glioblastoma; Glioma; Medulloblastoma; Meningioma; Neoplasms; Neuroectodermal Tumors; Neuroectodermal Tumors, Primitive; Oligodendroglioma; Optic Nerve Glioma; Rhabdoid Tumor and Spinal Cord Neoplasms.

The following examples are provided to further illustrate the invention. They are not to be construed to limit the scope of the embodiments or claims of the present invention.

### COMPARATIVE EXAMPLE:

### Comparative example 1

### An immediate release dosage formulations of veliparib

| | 40 mg Capsule | 80 mg Capsule |
|---|---|---|
| Veliparib (API) | 40 mg | 80 mg |
| Microcrystalline Cellulose (Filler) | 9 mg | 18 mg |
| Croscarmellose Sodium (First Disintegrant) | 14 mg | 28 mg |
| Hydroxypropyl Cellulose (Binder) | 4 mg | 8 mg |
| Colloidal silica (Glidant) | 3 mg | 6 mg |
| Magnesium Stearate (Lubricant) | 2 mg | 4 mg |

Veliparib free base, microcrystalline cellulose and croscarmellose sodium are mixed together for 15 min in a standard high shear mixer. Hydroxypropyl cellulose is dissolved in water. Granulation process is performed by adding granulation liquid to the dry mixture under stirring in a standard high shear mixer. The wet granules are sieved over 800 µm and are then dried in a fluidized bed drier at 40°C for 3 hours. Colloidal silica, Sodium starch glycolate and magnesium stearate are added to the mixture and the final blend is mixed for another 5 minutes. The mixture is filled in commercially available hard gelatin capsules to obtain an immediate release dosage form.

In order to test the release of the active substance - veliparib - from the dosage form, an in- vitro dissolution of the tablet is carried out by using the USP Paddle method at 50 rpm. (Dissolution test, USP 24 p.1941)

Medium: Acetate buffer pH 4.5

More than 80 -85 % drug is dissolved after 15minutes, more than 90% is dissolved after 30 minutes and 100% drug is dissolved after 60 minutes.

### EXAMPLES:

### Example 1:

### Extended release dosage form of veliparib

| Ingredients | Weight per Tablet |
|---|---|
| Tablet Core | |
| Veliparib free base crystalline (API) | 80.00 mg |
| Calcium hydrogen phosphate dihydrate | 104.50 mg |
| Hypromellose (gel forming agent) | 100.00 mg |
| Povidone (Binder) | 12.80 mg |
| Silica, Colloidal Anhydrous (Glidant) | 0.50 mg |
| Magnesium Stearate (Lubricant) | 2.20 mg |
| Core weight | 300 mg |

| Coating | |
|---|---|
| Hypromellose (gel forming agent) | 4.40 mg |
| Macrogol 6000 | 0.30 mg |
| Glycerol | 0.30 mg |
| Magnesium Stearate (Lubricant) | 0.03 mg |
| Titanium dioxide | 0.08 mg |

Purified water is heated to 40°C and povidone is added. The mixture is cooled to room temperature. (25±5°C). Veliparib free base, calcium phosphate, mixtures of HPMC of various grades (HPMC 15 mPa.s (cps), HPMC 15000 mPa.s (cps), HPMC 100000 mPa.s (cps)) are mixed together for 15 min in a standard high shear mixer. After sieving and blending, the granulation process is performed by spray granulation. The lubricant magnesium stearate is added to the granulate thus obtained and compressed into tablets.

### Coating:

The tablets are spray-coated in a tablet coating machine using an aqueous coating suspension of HPMC (5 mPa.s (cP)), Macrogol 6000 and high speed homogenized suspended titanium dioxide.

HPMC (15000 mPa.s (cP)) has a viscosity within the range of 11250-21000 mPa.s(cP) and substitution degree of methoxy groups of 19-24% by weight and hydroxypropoxy groups of 7-12% by weight. The viscosity values are given for 2% (w/w) aqueous solutions at 20°C.

In order to test the release of the active substance - veliparib - from the tablet, an in vitro dissolution of the tablet is carried out by using the USP Paddle method at 50 rpm. (Dissolution test, USP 24 p.1941)
Medium: Acetate buffer pH 4.5

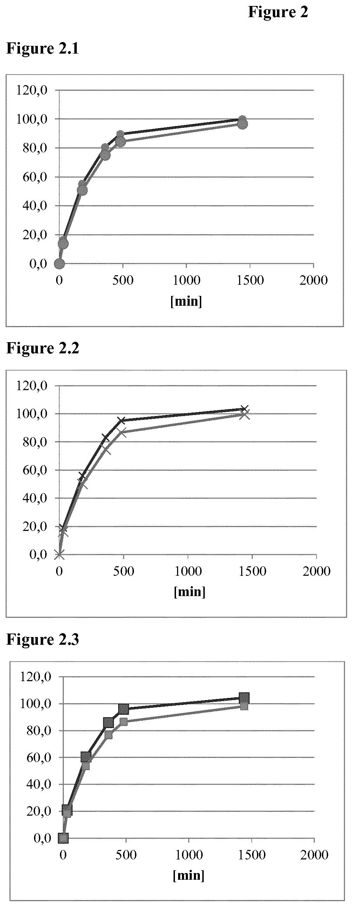

Figure 2 provides an In-vitro dissolution profile in the above medium.

### Example 2

### Extended release dosage form of Veliparib

| Ingredients | Weight per tablet |
|---|---|
| Tablet Core | |
| Veliparib (API) | 80.00 mg |
| Microcrystalline cellulose | 92.50 mg |
| Polyox WSR 301 NF | 120.00 mg |
| Povidone (Binder) | 12.80 mg |
| Silica, Colloidal Anhydrous (Glidant) | 2.50 mg |
| Magnesium Stearate (Lubricant) | 5.00 mg |
| Tablet core | 300 mg |

| Coating | |
|---|---|
| Hypromellose (gel forming agent) | 4.40 mg |
| Macrogol 6000 | 0.30 mg |
| Glycerol | 0.30 mg |
| Magnesium Stearate (Lubricant) | 0.03 mg |
| Titanium dioxide | 0.08 mg |

### Preparation:

i) mixing Veliparib with the gel-forming polymer polyethylene oxide and the release modifying agent, binders, lubricants, modifying agents and other excipients to obtain a powder mixture,
ii) forming the obtained powder mixture into a suitable solid dosage form and
iii) coating the obtained dosage form as in example 1.

### Example 3

### Extended release dosage form of Veliparib

| Ingredients | Weight per tablet |
|---|---|
| Tablet Core | |
| Veliparib (API) | 80.00 mg |
| Calcium hydrogen phosphate dihydrate | 104.50 mg |
| Methocel E10 M | 90.00 mg |
| Xanthan gum | 10.00 mg |
| Povidone (Binder) | 12.80 mg |
| Silica, Colloidal Anhydrous (Glidant) | 0.50 mg |
| Magnesium Stearate (Lubricant) | 2.20 mg |
| Core weight | 300 mg |

| Coating | |
|---|---|
| Hypromellose (gel forming agent) | 4.40 mg |
| Macrogol 6000 | 0.30 mg |
| Glycerol | 0.30 mg |
| Magnesium Stearate (Lubricant) | 0.03 mg |
| Titanium dioxide | 0.08 mg |

Processes for the manufacture of an extended release dosage form according to this example comprises following steps:
i) mixing veliparib with the gel-forming polymer which is combination of HPMC and xanthan gum and release modifying agent and mixing
ii) compressing the dry mixed mass into loose compacts,
iii) milling the compacts and mixing them with other excipients to obtain a powder mixture,
iv) forming the obtained dry powder mixture into a suitable solid dosage form and
v) coating the obtained dosage form as in example 1.

### Example 4

The calculated blood levels from administration of the extended release dosage form of example 1 is determined in two experiments.

Example 4 A - In this experiment 3 tablets containing 80 mg veliparib each prepared in accordance with example 1 are administered twice a day, 12h apart, to meet a daily dose of 480 mg. A plot displaying the pharmacokinetic behaviour is obtained by plotting the blood plasma concentration of veliparib against time. The Cₘₐₓ/Cₘᵢₙ ratio for veliparib is not more than 3. After 7 days Cₘₐₓ and Cₘᵢₙ are both close to their respective steady state concentrations. Cₘᵢₙ is about 0.5 µg/ml

Example 4 B - In this experiment 4 tablets containing 80 mg veliparib each prepared in accordance with example 1 are administered twice a day, 12h apart, to meet a daily dose of 640 mg. A plot displaying the pharmacokinetic behaviour is obtained by plotting the blood plasma concentration of veliparib against time. The Cₘₐₓ/Cₘᵢₙ ratio for veliparib is not more than 3.

After 7 days Cₘₐₓ and Cₘᵢₙ are both close to their respective steady state concentrations. Cₘᵢₙ is about 0.6 µg/ml

### Example 5

In this experiment 10 tablets containing 40 mg veliparib each prepared in accordance with Comparative example 1 are administered twice a day, 12h apart, to meet a daily dose of 800 mg. A plot displaying the pharmacokinetic behaviour of veliparib is obtained by plotting the blood plasma concentration of veliparib against time (Figure 1). Cₘᵢₙ is around 0.5 µg/ml whereas Cₘₐₓ almost reaches 3.5 µg/ml. The Cₘₐₓ/Cₘᵢₙ ratio for veliparib is almost 7.

## Claims

1. An extended release oral dosage form comprising 2-((R)-2-Methylpyrrolidin-2-yl)-1H-benzimidazole-4-carboxamide (veliparib) and a gel-forming polymer for the treatment of cancer, wherein the extended release dosage form is to be administered twice daily.

2. The extended release oral dosage form according to claim 1 further comprising pharmaceutically acceptable excipients selected from binders, lubricants, release modifying agents and flow condition agents.

3. The extended release oral dosage form according to any one of claims 1 and 2, wherein veliparib is crystalline veliparib free base.

4. The extended release oral dosage from according to any one of claims 1 to 3, wherein the gel-forming polymer is selected from the group of hydroxymethyl cellulose, hydroxypropyl cellulose, hydroxyethyl cellulose, hydroxypropyl methylcellulose, methyl cellulose, ethyl cellulose, polyvinylpyrrolidone, polyethylene glycols, polyethylene oxide and poloxamers.

5. The extended release oral dosage form according to any one of claims 1 to 4, wherein the gel-forming polymer is HPMC.

6. The extended release oral dosage form according to claim 5, wherein HPMC is a mixture of HPMC having a substitution degree of methoxy groups of said HPMC from 19 to 30% by weight, and a substitution degree of hydroxypropoxy groups of said HPMC from 4 to 12% by weight, and wherein said HPMC is a mixture of HPMC of various grades comprising viscosities 15 mPa.s (cps), 15000 mPa.s (cps), 100000 mPa.s (cps).

7. The extended release oral dosage form according to any one of claims 1 to 6, wherein the dosage form is coated.

8. The extended release oral dosage form according to claim 7, wherein the coating layer comprises a polymer such as ethyl cellulose, hydroxypropyl cellulose, polyethylene glycol, low viscosity HPMC, acrylic and methacrylic esters, ethyl acrylate and methyl methacrylate or mixtures thereof.

9. The extended release oral dosage form according to any one of claims 1 to 8, which is a tablet.

10. The extended release oral dosage form according to claim 9, which is a film coated tablet.

11. The extended release oral dosage form according to any one of claims 1 to 10, wherein the amount of veliparib free base in the dosage form is from 20mg to 200mg.

12. Processes for the manufacture of an extended release dosage form according to claim 1 comprising the steps:
Ai)
a. mixing the veliparib with the gel-forming polymers, release modifying agent and optionally binders and other excipients,
b. sieving and blending the mixture,
c. spray granulating said mixture,
d. optionally drying the obtained granulate,
e. adding lubricant to the granulate of step d) and sieving,
f. compressing the granulate powder mass of step e),
Aii) forming the obtained dry powder mixture into a suitable solid dosage form and
Aiii) coating the obtained dosage form.

## Patentansprüche

1. Orale Dosierungsform mit verlängerter Freisetzung, umfassend 2-((R)-2-Methylpyrrolidin-2-yl)-1H-Benzimidazol-4-carboxamid (Veliparib) und ein gelbildendes Polymer zur Behandlung von Krebs, wobei die Dosierungsform mit verlängerter Freisetzung zweimal täglich zu verabreichen ist.

2. Die orale Dosierungsform mit verlängerter Freisetzung nach Anspruch 1 umfassend ferner pharmazeutisch akzeptable Hilfsstoffe, ausgewählt aus Bindemitteln, Schmiermitteln, die Freisetzung modifizierenden Mitteln und Fließkonditionierungsmitteln.

3. Die orale Dosierungsform mit verlängerter Freisetzung nach einem der Ansprüche 1 und 2, wobei Veliparib eine kristalline Veliparib-freie Base ist.

4. Die orale Dosierungsform mit verlängerter Wirkstofffreisetzung nach irgendeinem der Ansprüche 1 bis 3, wobei das gelbildende Polymer ausgewählt ist aus der Gruppe von Hydroxymethylcellulose, Hydroxypropylcellulose, Hydroxyethylcellulose, Hydroxypropylmethylcellulose, Methylcellulose, Ethylcellulose, Polyvinylpyrrolidon, Polyethylenglykolen, Polyethylenoxid und Poloxameren.

5. Die orale Dosierungsform mit verlängerter Freisetzung nach irgendeinem der Ansprüche 1 bis 4, wobei das gelbildende Polymer HPMC ist.

6. Die orale Dosierungsform mit verlängerter Freisetzung nach Anspruch 5, wobei HPMC eine Mischung aus HPMC mit einem Substitutionsgrad von Methoxygruppen des HPMC von 19 bis 30 Gew.-% und einem Substitutionsgrad von Hydroxypropoxygruppen des HPMC von 4 bis 12 Gew.-% ist, und wobei das HPMC eine Mischung aus HPMC verschiedener Grade umfassend Viskositäten von 15 mPa.s (cps), 15000 mPa.s (cps), 100000 mPa.s (cps) ist.

7. Die orale Dosierungsform mit verlängerter Freisetzung nach irgendeinem der Ansprüche 1 bis 6, wobei die Dosierungsform beschichtet ist.

8. Die orale Dosierungsform mit verlängerter Freisetzung nach Anspruch 7, wobei die Beschichtungsschicht ein Polymer wie Ethylcellulose, Hydroxypropylcellulose, Polyethylenglykol, niedrigviskoses HPMC, Acryl- und Methacrylsäureester, Ethylacrylat und Methylmethacrylat oder Mischungen davon umfasst.

9. Die orale Dosierungsform mit verlängerter Freisetzung nach irgendeinem der Ansprüche 1 bis 8, welche eine Tablette ist.

10. Die orale Dosierungsform mit verlängerter Freisetzung nach Anspruch 9, welche eine filmbeschichtete Tablette ist.

11. Die orale Dosierungsform mit verlängerter Freisetzung nach irgendeinem der Ansprüche 1 bis 10, wobei die Menge der freien Veliparib-Base in der Dosierungsform von 20 mg bis 200 mg beträgt.

12. Verfahren zur Herstellung einer Dosierungsform mit verlängerter Freisetzung nach Anspruch 1, umfassend die Schritte:
Ai)
a. Mischen des Veliparibs mit den gelbildenden Polymeren, Freisetzungsmodifizierungsmittel und gegebenenfalls Bindemitteln und anderen Hilfsstoffen,
b. Sieben und Mischen der Mischung,
c. Sprühgranulieren dieser Mischung,
d. gegebenenfalls Trocknen des erhaltenen Granulats,
e. Zugabe von Schmiermittel zum Granulat von Schritt d) und Sieben,
f. Verpressen der Granulatpulvermasse von Schritt e),
Aii) das erhaltene trockene Pulvergemisch in eine geeignete feste Dosierungsform bringen und
Aiii) Beschichten der erhaltenen Dosierungsform.

## Revendications

1. Forme posologique orale à libération prolongée comprenant le 2-((R)-2-méthylpyrrolidin-2-yl)-1H-benzimidazole-4-carboxamide (véliparib) et un polymère filmogène pour le traitement du cancer, dans laquelle la forme posologique à libération prolongée est à administrer deux fois par jour.

2. Forme posologique orale à libération prolongée selon la revendication selon la revendication 1 comprenant en outre des excipients pharmaceutiquement acceptables sélectionnés parmi les liants, les lubrifiants, les agents modificateurs de libération et les agents d'écoulement.

3. Forme posologique orale à libération prolongée selon l'une quelconque des revendications 1 et 2, dans laquelle le véliparib est une base libre de véliparib cristalline.

4. Forme posologique orale à libération prolongée selon l'une quelconque des revendications 1 à 3, dans laquelle le polymère filmogène est sélectionné à partir du groupe constitué par l'hydroxyméthylcellulose, l'hydroxypropylcellulose, l'hydroxyéthylcellulose, l'hydroxypropylméthylcellulose, la méthylcellulose, l'éthylcellulose, la polyvinylpyrrolidone, les polyéthylène glycols, l'oxyde de polyéthylène et les poloxamères.

5. Forme posologique orale à libération prolongée selon l'une quelconque des revendications 1 à 4, dans laquelle le polymère filmogène est l'HPMC.

6. Forme posologique orale à libération prolongée selon la revendication 5, dans laquelle l'HPMC est un mélange d'HPMC ayant un degré de substitution de groupes méthoxy de ladite HPMC de 19 à 30 % en poids, et un degré de substitution de groupes hydroxypropoxy de ladite HPMC de 4 à 12 % en poids, et dans laquelle ladite HPMC est un mélange d'HPMC de diverses qualités comprenant des viscosités de 15 mPa.s (cps), 15 000 mPa.s (cps), 100 000 mPa.s (cps).

7. Forme posologique orale à libération prolongée selon l'une quelconque des revendications 1 à 6, dans laquelle la forme posologique est enrobée.

8. Forme posologique orale à libération prolongée selon la revendication 7, dans laquelle la couche d'enrobage comprend un polymère tel que l'éthylcellulose, l'hydroxypropylcellulose, le polyéthylène glycol, l'HPMC à faible viscosité, les esters acryliques et méthacryliques, l'acrylate d'éthyle et le méthacrylate de méthyle ou des mélanges de ceux-ci.

9. Forme posologique orale à libération prolongée selon l'une quelconque des revendications 1 à 8, qui est un comprimé.

10. Forme posologique orale à libération prolongée selon la revendication 9, qui est un comprimé pelliculé.

11. Forme posologique orale à libération prolongée selon l'une quelconque des revendications 1 à 10, dans laquelle la quantité de base libre de véliparib dans la forme posologique est de 20 mg à 200 mg.

12. Procédés pour la fabrication d'une forme posologique à libération prolongée selon la revendication 1 comprenant les étapes de :
Ai)
a. mélange du véliparib avec les polymères filmogènes, l'agent modificateur de libération et facultativement des liants et d'autres excipients,
b. tamisage et malaxage du mélange,
c. granulation par pulvérisation dudit mélange,
d. facultativement séchage du granulat obtenu,
e. ajout de lubrifiant au granulat de l'étape d) et tamisage,
f. compression de la masse de poudre de granulat de l'étape e),
Aii) mise en forme dudit mélange de poudre sèche obtenu en une forme posologique solide adéquate et
Aiii) enrobage de la forme posologique obtenue.
